# EUROPEAN PATENT SPECIFICATION

(11) **EP 1 792 621 B1**
(45) Date of publication and mention of the grant of the patent: **04.04.2012**
(21) Application number: 05111534.3
(22) Date of filing: 30.11.2005
(51) Int. Cl.: A61K 31/727, A61K 9/00

(54) **Orally administrable heparin derivatives**
Oral verabreichbare Heparinderivate
Dérivés de l'héparine administrables par voie orale

(43) Date of publication of application: 06.06.2007
(73) Proprietor: Istituto di Ricerche Chimiche e Biochimiche "G. Ronzoni", 20133 Milano (IT)
(72) Inventor: Torri, Giangiacomo, 20133, Milano (IT); Naggi, Annamaria, 20025, Legnano (Mi) (IT)
(74) Representative: Serravalle, Marco

(56) References cited:
- US-A- 5 013 724
- US-A1- 2005 020 539
- JAQUES, L. B. ET AL: "Evidence from endothelium of gastric absorption of heparin and of dextran sulfates 8000" JOURNAL OF LABORATORY AND CLINICAL MEDICINE , 117(2), 122-30 CODEN: JLCMAK; ISSN: 0022-2143, 1991, XP008061620
- MAJURU, SHINGAI: "Advances in the oral delivery of heparin from solid dosage forms using emisphere's eligen oral drug delivery technology" DRUG DELIVERY TECHNOLOGY , 4(8), 84,86-89 CODEN: DDTRAW; ISSN: 1537-2898, 2004, XP008061644

## Description

### State of the Art

Heparin is a well known antithrombotic drug normally administered by intravenous or subcutaneous route. In fact, it is well known that oral bioavailability of unfractionated heparin (UHF) is negligible. Several studies has been performed in recent years to find ways to increase oral bioavailability of heparin.

A.K. Larsen et al. "Oral heparin results in the appearance of heparin fragments in the plasma rats" Proc Natl Acad Sci USA, 1986, 83(9), 2964-2968, disclose that oral administration of radioactive heparin results in continuous release of radioactive material into the bloodstream for at least 12 h. The radioactive material is identified as oligo-, di, and monosaccharides. This study confirms that only short chains are metabolized by oral route. WO 00/48589 and WO 01/34114 propose a method for improving intestinal absorption of heparin based on combining heparin with the carrier molecule sodium N-(8[2-hydroxybenzoyl]amino)caprylate.

Y Jiao et al. "In vitro and in vivo evaluation of oral heparin-loaded polymeric nanoparticles in rabbits" Circulation, 2002, 105(2), 230-5, disclose another carrier suitable for improving oral administration of heparin: polymeric nanoparticles prepared with biodegradable poly-epsilon-caprolactone and poly(lactic-co-glycolic acid) and positively charged polymers.

WO 03/049721 discloses a controlled-release formulation consisting of a multimatrix structure comprising a matrix comprising amphiphilic compounds and lipophilic compounds in which heparin is at least partially englobated, and an outer hydrophilic matrix, in which the first matrix is dispersed.

Another approach to improve bioavalaibility of orally administered heparin is by chemical modification of heparin itself.

EP 496233 discloses supersulfated heparin having Mw comprised between 2000 and 9000 Da (ssLMWH) for use in oral administration. In a preferred embodiment, supersulfated heparin is administered by gastro-resistant capsules.

US 5,013,724 discloses N-acetylated oversulfated low molecular weight heparin. Although the patent does not provide any in vivo data of intestinal absorption, it mentions inter alia the oral administration of these oligosaccharides by gastro-resistant capsules, tablets, lozenges, pills or liposomes.

### Summary of the invention

It is the object of the present invention the provision of new compositions for oral administration comprising a N-acetylated oversulfated heparin, wherein the composition is not in a gastro-resistant capsule, tablet, lozenge, pill or liposome.

In fact, it has been found that N-acetylated supersulfated heparin (NASSH) is capable of transiting the gastric section without major degradation and can be absorbed in the intestinal tract resulting in a high antithrombotic activity.

### Detailed description of the invention

The heparin derivatives for use in the present invention are characterized by the following parameters: percentage of N-acetylation >50%, preferably higher than 70%, degree of sulfation >2.5, preferably higher than 2.8, most preferably higher than 3, weight average molecular weight comprised between 1,600 and 10,000.

It was totally unexpected in view of the prior art that the N-acetylated oversulfated heparin of the present invention maintains the characteristics of absorption in the intestinal tract while having a lower degree of sulphation than ssLMWH; it is even more surprising that it is possible to administer NASSH orally without protecting it with a gastro-resistant capsule and without the use of any carrier system such as liposomes and polymeric nanoparticles prepared with biodegradable poly-epsilon-caprolactone and poly(lactic-co-glycolic acid) and positively charged polymers, or carrier molecules such as sodium N-(8[2-hydroxybenzoyl]amino)caprylate. Without being bound by any theory, it appears that the NASSH presents a balance of stability and bioavailability which makes it very suitable for oral administration.

The molecular weight of the NASSH useful in the present invention depends on the type of application. In fact, it is known that the reduction of Mw of heparin normally leads to a loss of active sites for antithrombotic activity. Thus, when considering the use of heparin as an antithrombotic drug, it is preferred to prepare a NASSH with an average molecular weight by weight comprised between 4,000 and 8,000.

NASSH according to the invention can be prepared according to several routes. Any process which combines a depolymerization step, a sulfation step and a N-acetylation step in any order is suitable for the preparation of NASSH for use in the present invention as long as the final supersulfated N-acertylated heparin fulfils the following parameters: percentage of N-acetylation >50%, degree of sulfation >2.5 and Mw comprised between 1,600 and 10,000.

N-acetylation is well known in the art. To prepare N-acetylated heparin, heparin is first N-desulfated by treatment either with aqueous acid solution or with a mixture dimethylsulfoxide (DMSO)/water or methanol at high temperature. It is also possible, by dosing the reaction conditions, to obtain only a partly N-desulfated heparin. N-desulfated heparin is afterwards N-acetylated e.g. by reaction with acetic anhydride in aqueous base.

Sulfation can be performed in several ways. One possible way of sulfating heparin is by dissolving it in a dipolar aprotic solvent such as DMSO, dimethylformamide (DMF), formamide, hexamethylenphosphotriamide (HMPT) or acetonitrile and contacting it with a sulfating agent such as sulfur trioxide complex with an organic base, or chlorosulfonic acid in pyridine.

As an example, heparin can be first N-acetylated and then supersulfated by using a sulfuric acid/chlorosulfonic acid mixture. Depending on the reaction time and temperature the reaction can lead to a depolymerization reaction as well. Thus, according to the reaction conditions, a supersulfated heparin having Mw comprised between 1,600 and 10,000 is obtained

If the sulfation method does not lead to depolymerization of heparin, it is necessary to apply a depolymerization step. Depolymerization of heparin can be obtained by chemical or physical treatment. Amongst the most commonly used chemical processes we can mention depolymerization by nitrous acid (EP 0 014 184, EP 0 037 319, EP0 076 279, EP 0 394 971 EP 0 623 629,) alkaline depolymerization (DE4121115) ss elimination from an heparin ester (EP 0 040 144, US 5,389,618), périodate (EP 0 287 477),sodium borohydride (EP 0 347 588, EP 0 380 943), ascorbique acid (US 4,533,549) ; hydrogen peroxyde (US 4,629,699, US 4,791,195), ammonium hydroxide starting from a quaternary salt of heparin (US 4,981,955), alkali metal hydroxide (EP 0 380 943, EP 0 347 588) or by enzymatic route (EP 0 064 452, US 4,396,762, EP 0 244 235, EP0 244 236 ; US 4,826,827 ; US 3,766,167) and by Cu²⁺/hydrogen peroxide (WO 90/04607). Amongst the physical processes, depolymerization by gamma rays (WO 2003/076474), by electron beam (WO 2004/000886), and by UV irradiation (WO 2004/099256).

### EXPERIMENTAL SECTION

Molecular mass (Mw) was determined by size exclusion chromatography The average molecular weights (Mw), in Da, and polydispersion (D) were measured by GPC-HPLC on a Viscotex instrument equipped with VE1121 pump, Rheodyne valve 100 µl and TDA (Triple Detector Array) 302 equipped with R.I., viscosimeter and 90° light scattering systems. Two 300 x 7.8 mm TSK GMPWXL Viscotek columns were used, with 0.1M NaNO3 as eluent (flow 0.6 ml/min). Samples were dissolved in the eluent solution at the concentration of 15 mg/ml. Bertini, S., Bisio, A., Torri, G., Bensi, D., and Terbojevich, M. Molecular weight determination of heparin and dermatan sulfate by size exclusion chromatography with a triple detector array. Biomacromolecules, 2005; 6: 168-173 .
Anti Xa activity was determined according to the method described in European Pharmacopoeia 4^{th} ed.: 2.2.30 and 2.2.46 for chromatography techniques and 01/2002:0828 p.1297 for method.
Anti coagulant activity was determined according to the method described in European Pharmacopoeia 4^{th} ed.: 2.7.5 pg 168.
Sulfate and carboxylate ions were determined by the average sulfation degrees (S.D.), calculated by addition of the molar fraction of sulfate groups for disaccharidic unit evaluated by integration of NMR signals Guerrini M, Naggi A, Guglieri S, Santarsiero R, Torri G. Complex glycosaminoglycans: profiling substitution patterns by two-dimensional nuclear magnetic resonance spectroscopy. Anal Biochem 2005;337: 35-47..

### Example 1 (G 3958): oversulfated low molecular weight N-acetylated heparin prepared applying sulfuric acid/chlorosulfonic acid mixture on a N-acetylated heparin.

15 g of heparin sodium salt (code G3000) were dissolved in water and the solution was eluted from a column of Amberlite IR-120 (H+) resin. After adjusting the pH to 7 by addition of pyridine, the solution was evaporated under reduced pressure. The resulting pyridine salt was dissolved in Me₂SO/H₂O 95/5 v/v solution (450 ml). The solution was stirred for 48 h at room temperature. At the end of the reaction, the solution was diluted with an equal volume of distilled water and the pH was adjusted to about 9 by addition of NaOH 1 M.

The sample was precipitated with 4 volumes of EtOH saturated with sodium acetate, recovered by filtration, dissolved in distilled water and dialyzed against distilled water in membranes (cut-off 2000 D) for three days . By evaporation under reduced pressure, a N-desulfated sodium heparin (code G3852) was obtained.

The obtained sample was dissolved in 200 ml of saturated sodium bicarbonate solution and 20 ml of acetic anhydride were added at 0°C (if necessary further additions of saturated sodium bicarbonate were made to maintain the pH at 8 or above). The solution was stirred at 0 - 4 °C for 4h and the product was precipitated with 4 volumes of EtOH saturated with sodium acetate. The sample, recovered by filtration, was dissolved in distilled water and dialyzed against distilled water in membranes (cut-off 2000 D) for three days. By evaporation under reduced pressure, 11,5 g of a N-acetylated sodium heparin (code G3875) were obtained in 77 % yield by weight.

3g of G3875 were added to a mixture of 60 ml of 98% sulphuric acid and 30 ml of 95% chlorosulfonic acid. The mixture was stirred for 1 hour at room temperature and then poured into 500 ml of cold diethyl ether (-40°). The precipitate was filtered and washed with cold diethyl ether. The obtained product was dissolved in water, neutralized with 1 N sodium hydroxide and dialysed against distilled water in membranes (cut-off 1000 D) for three days. By evaporation under reduced pressure, 1.7g of a depolymerised and supersulfated N-Acetyl heparin (code G3958) were obtained in 67% yield by weight, having the following characteristics: Mw 6890, NAcetyl > 90% , degree of sulfation 3.8

### Example 2 (G 5134): oversulfated low molecular weight N-acetylated heparin prepared applying sulfuric acid/chlorosulfonic acid mixture on a N-acetylated heparin.

3g of G3875 (NAc-heparin) were added to a mixture of 60 ml of 98% sulphuric acid and 30 ml of 95% chlorosulfonic acid. The mixture was stirred for 1 hour at room temperature and then poured into 500 ml of cold diethyl ether (-40°). The precipitate was filtered and washed with cold diethyl ether. The obtained product was dissolved in water, neutralized with 1 N sodium hydroxide and dialysed against distilled water in membranes (cut-off 1000 D) for three days. By evaporation under reduced pressure, 1.7g of a depolymerised and supersulfated N-Acetyl heparin (code G5134) were obtained in 75% yield by weight, having the following characteristics: Mw 7100, NAcetyl > 90%, degree of sulfation 3.8

### Example 3 (G3897d): oversulfated low molecular weight partially N-acetylated heparin obtained through oversulfation reaction under standard conditions from a LMW(50%) NAc-H.

A solution of 10g of heparin sodium salt (code G3000) in 250 ml of water was cooled at 4°C and 163 mg of NaNO₂ were added. The pH was adjusted to 2 by addition of HCl (4%) and the solution was stirred for 17'. At the end of this time the reaction was stopped by addition of NaOH (till the pH was equal to 7) and a solution of NaBH₄ (1g in 10 ml) was added and the mixture was stirred at room temperature for 3 h. At the end of the reduction the pH was adjusted to 4 by addition of HCl, the solution was stirred for 10' and finally neutralized with NaOH 1M. The solution volume was reduced to 100 ml and the sample was precipitated with 4 volumes of EtOH saturated with sodium acetate, recovered by filtration and dissolved in distilled water; by evaporation under reduced pressure a low molecular weight heparin (code G3813) was obtained.

A low molecular weight heparin (G3813), prepared according with procedure described above, was dissolved in water and the solution was eluted from a column of Amberlite IR-120 (H+) resin. After adjusting the pH to 8 by addition of pyridine, the solution was evaporated under reduced pressure. The resulting pyridine salt was dissolved in Me₂SO/H₂O 95/5 v/v solution (400 ml). The solution was stirred for 3 h at room temperature. At the end of the reaction, the solution was diluted with an equal volume of distilled water, the pH was adjusted to about 9 by addition of a solution of NaHCO₃ and the partial N desulfated low molecular weight heparin (code G3831) was recovered by evaporation under reduced pressure. The obtained sample was dissolved in 300 ml of saturated sodium bicarbonate solution and 12 ml of acetic anhydride were added at 0°C (if necessary further additions of saturated sodium bicarbonate were made to maintain the pH at 8 or above).

The solution was stirred at 0 - 4 °C for 4 h and the product was precipitated with 4 volumes of EtOH saturated with sodium acetate. The sample, recovered by filtration, was dissolved in distilled water and dialyzed against distilled water in membranes (cut-off 1000 D) for three days. By evaporation under reduced pressure, a partial N Acetylated low molecular weight heparin (code G3924) was obtained.

The sample was dissolved in H₂O and the solution was eluted from a column of Amberlite IR-120 (H+) resin. After adjusting the pH to 5.5 by addition of pyridine, the solution was evaporated under reduced pressure. The resulting pyridine salt was dissolved in dry DMF (400 ml) and an excess of Py.SO₃ complex, dissolved in dry DMF (36 mg/100ml), was added and the mixture was stirred for 4 h at 55°C. At the end of the reaction, the solution was diluted with an equal volume of distilled H₂O, the pH was adjusted to about 9 by addition of NaOH 2 M and the product was precipitated with 6 volumes of EtOH saturated with sodium acetate. The sample, recovered by filtration, was dissolved in distilled water and dialyzed against distilled water in membranes (cut-off 1000 D) for three days.

By evaporation under reduced pressure, G3867 was obtained. The sample spectrum showed that it was partially N-desulfated. For this reason it was dissolved in 200 ml of H₂O saturated with NaHCO₃ and 13g of trimethylamine SO₃ complex (TMA.SO₃) were added at 55°C (if necessary further additions of saturated sodium bicarbonate were made to maintain the pH at 8 or above). The solution was stirred at 55°C for 18 h and then neutralized; the product was precipitated with 4 volumes of EtOH saturated with sodium acetate, recovered by filtration, dissolved in distilled water and dialyzed against distilled water in membranes (cut-off 1000 D) for three days. By evaporation under reduced pressure, a partly N-Acetylated oversulfated low molecular weight sodium heparin (G3987) was obtained as a powder having the following characteristics MW 6250 D, % NAc 74, degree of sulfation SO₃⁻/COO⁻= 2.9.

### Example 4 (G3965): oversulfated low molecular weight N-acetylated heparin obtained through O-sulfation reaction under standard conditions from a LMWNAcH.

A solution of 6 g of heparin sodium salt (code G3000) in 150 ml of water was cooled at 4°C and 156 mg of NaNO₂ were added. The pH was adjusted to 2 by addition of HCI (4%) and the solution was stirred for 17'. At the end of this time the reaction was stopped by addition of NaOH (till the pH was equal to 7) and a solution of NaBH₄ (1g in 10 ml) was added and the mixture was stirred at room temperature for 3 h. At the end of the reaction the pH was adjusted to 4 by addition of HCl, the solution was stirred for 10'and finally neutralized with NaOH 1M. The solution volume was reduced to 100 ml and the sample was precipitated with 4 volumes of EtOH saturated with sodium acetate, recovered by filtration and dissolved in water; by evaporation under reduced pressure, a low molecular weight sodium heparin (code G3910) was obtained.

Low molecular weight heparin (G3910 - 5 g), prepared according with procedure described above, was dissolved in water and the solution was eluted from a column of Amberlite IR-120 (H+) resin. After adjusting the pH to 7 by addition of pyridine, the solution was evaporated under reduced pressure. The resulting pyridine salt was dissolved in Me₂SO/H₂O 95/5 v/v solution (200 ml). The solution was stirred for 48 h at room temperature. At the end of the reaction, the solution was diluted with an equal volume of distilled water, the pH was adjusted to about 9 by addition of a solution of NaHCO₃ and N-desulfated low molecular weight heparin (code G3918) was recovered by evaporation under reduced pressure. The obtained sample was dissolved in 100 ml of saturated sodium bicarbonate solution and 20 ml of acetic anhydride were added at 0°C (if necessary further additions of saturated sodium bicarbonate were made to maintain the pH at 8 or above).

The solution was stirred at 0 - 4 °C for 4 h and the product was precipitated with 4 volumes of EtOH saturated with sodium acetate. The sample, recovered by filtration, was dissolved in distilled water and dialyzed against distilled water in membranes (cut -off 1000 D) for three days. By evaporation under reduced pressure a N-Acetylated low molecular weight sodium heparin (code G3924) was obtained.

The sample was dissolved in H₂O and the solution was eluted from a column of Amberlite IR-120 (H+) resin. After adjusting the pH to 5.5 by addition of pyridine, the solution was evaporated under reduced pressure. The resulting pyridine salt was dissolved in dry DMF (300 ml) and an excess of Py.SO₃ complex, dissolved in dry DMF (26 mg/100ml), was added and the mixture was stirred for 18 h at 55°C. At the end of the reaction, the solution was diluted with an equal volume of distilled H₂O, the pH was adjusted to about 9 by addition of NaOH 2 M and then the product was precipitated with 6 volumes of EtOH saturated with sodium acetate. The sample, recovered by filtration, was dissolved in distilled water and dialyzed against distilled water in membranes (cut -off 1000 D) for three days. By evaporation under reduced pressure 1.4g of G3928 were obtained in 30% yield by weight. The sulfation procedure was repeated and 1.2g of G3965 were obtained having the following characteristics: molecular weight 6250 D, % NAc 100, sulfation degree SO₃⁻/COO⁻ =3.3

### Comparative Example 5 (G 3919 A): preparation of oversulfated low molecular weight heparin prepared according to EP 0 116 80 (sulfuric acid/chlorosulfonic acid mixture).

To a mixture of 30 ml of 98% sulphuric acid and 15 ml of 95% chlorosulfonic acid were added 1.5g of heparin (code number G3000) obtained by LDO (Trino Vercellese, Italy) having MW 19600 and SO₃⁻/COO⁻2.1. The mixture was stirred for 1 hour at room temperature, poured into 250 ml of cold diethyl ether (-40°) and the obtained precipitate was filtered and washed with cold diethyl ether. The resulting product was dissolved in water, neutralized with 1 N sodium hydroxide and dialysed against distilled water in membranes (cut-off 1000 D) for three days. By slow evaporation under reduced pressure 1.2 g of depolymerised and supersulfated sodium heparin (code G3919a) were obtained in 70% yield by weight, as powder having the following characteristics :Mw 8800 D, NAc 6%, degree of sulfation SO₃⁻/COO⁻ =4.5

### IN VITRO STUDIES

The products obtained in examples 1-5 has been characterized as follows:
1) Effect on PTTa (Tab 1)
2) Effect on TcT (Tab 2)
3) Effect on anti Xa activity (Tab 3)
4) Ecarin clotting time (Tab 4)

As reference compounds unfractionated heparins (Liquemin, G3000) and a low molecular weight heparin (enoxaparin,) were tested. In some selected experiments dermatan sulfate was used as reference compound.

Studies were carried out in human normal plasma (plasma pool from 20 healthy volunteers); compounds under testing were added to the plasma and the tests were carried out immediately according to a standard method (S. Momi, M. Nasimi, M. Colucci, G.G. Nenci, P. Gresele, Haematologica 2001, 86: 297-302.).

**Tab. 1 Effect on PTTa (sec)**

| | **0.1 µg/ml** | **0.5 µg/ml** | **1.0 µg/ml** | **2.5 µg/ml** | **5 µg/ml** | **10 µg/ml** | **25 µg/ml** | **50 µg/ml** |
|---|---|---|---|---|---|---|---|---|
| Example 1 | 28.13 | 29.03 | 30.23 | 33.33 | 38.77 | 52.00 | 99.78 | 300 |
| Example 3 | 28.57 | 29.33 | 30.5 | 34.13 | 40.23 | 51.28 | 250.75 | 300 |
| Example 4 | 28.70 | 29.50 | 30.97 | 35.03 | 41.27 | 58.03 | 300 | 300 |
| Comp. Ex. 5 | 28.03 | 29.33 | 31.37 | 37.37 | 48.27 | 77.9 | 300 | 300 |
| Enoxa | 32.73 | 33.87 | 35.73 | 44.67 | 58.77 | 89.13 | 300 | 300 |
| Liquemin | 29.63 | 37.37 | 46.27 | 154.87 | 300 | 300 | 300 | 300 |
| G3000 | - | - | - | - | - | 300 | 300 | 300 |

**Tab. 2 Effect on TcT (sec)**

| | **0.1 µg/ml** | **0.5 µg/ml** | **1 µg/ml** | **2.5 µg/ml** | **5 µg/ml** | **10 µg/ml** | **25 µg/ml** | **50 µg/ml** |
|---|---|---|---|---|---|---|---|---|
| Example 1 | 16 | 17,1 | 18.4 | 23.3 | 29.4 | 38 | 167 | 167 |
| Example 3 | 15.7 | 16.4 | 17.9 | 22.4 | 26.9 | 3.3 | 167 | 167 |
| Example 4 | 16.4 | 17.2 | 18.6 | 21.8 | 27.2 | 36.1 | 167 | 167 |
| Comp. Ex. 5 | 16.3 | 19.8 | 24.9 | 34.6 | 47.5 | 167 | 167 | 167 |
| Enoxa | 15.2 | 15.6 | 17.9 | 30.1 | 167 | 167 | 167 | 167 |
| Liquemin | 13.9 | 20.3 | 167 | 167 | 167 | 167 | 167 | 167 |
| G3000 | - | - | - | - | - | 167- | 167 | 167 |

All the different heparin derivatives induced a dose-dependent prolongation of the thrombin clotting time (TcT) that became immeasurable at the highest dose tested. Here too, G3958 and G3987d showed a lesser prolongation of the TcT that became uncoagulable only at the dose of 50 µg/ml. Liquemin and G3000 prolonged the TcT almost at all doses tested while enoxaparin showed a maximal effect starting from 5 µg/ml.

All the heparin derivatives increased anti Xa activity dose-dependently, although considerably less than the reference compound.

**Tab. 3 Effect on anti Xa activity (U/ml)**

| | 0.1 µg/ml | 0.5 µg/ml | 1 µg/ml | 2.5 µg/ml | 5 µg/ml | 10 µg/ml | 25 µg/ml | 50 µg/ml |
|---|---|---|---|---|---|---|---|---|
| Example 1 | 0.02 | 0.04 | 0.05 | 0.07 | 0.12 | 0.23 | 0.25 | 0.35 |
| Example 3 | 0.07 | 0.08 | 0.14 | 0.19 | 0.11 | 0.13 | 0.30 | 0.33 |
| Comp. Ex. 5 | 0.07 | 0.06 | 0.07 | 0.09 | 0.15 | 0.24 | 0.49 | 0.85 |
| Enoxa | 0.05 | 0.05 | 0.08 | 0.22 | 0.28 | 1.23 | 2.64 | 4.63 |
| Liquemin | 0.00 | 0.08 | 0.27 | 0.66 | 0.80 | 2.50 | 3.12 | 4.40 |
| UFH | - | - | - | - | - | 2.5 | >3.2 | >3.2 |

Ecarin clotting time (ECT) has been proposed as a test for the specific monitoring of antithrombin drugs, such as hirudin, argatroban, and hirulog. Aqueous reagent and dry chemistry technology have become available for ECT monitoring of antithrombin agents.

These agents activate heparin cofactor II (HCII) and primarily mediate their effects by inhibiting thrombin. Given the low anti Xa activity of some of these agents their effects on ECT was measured in human plasma incubated with different doses of the heparin derivatives in order to detect an HCII-mediated effect. Dermatan sulphate, a known HCII activator, was used as a reference compound. The sensitivity of ECT was adjusted by varying the concentration of the ecarin reagent.

**Tab. 4 Ecarin clotting time (sec)**

| | 10 µg/ml | 15 µg/ml | 20 µg/ml | 25 µg/ml | 30 µg/ml | 40 µg/ml | 50 µg/ml |
|---|---|---|---|---|---|---|---|
| Dermatan | 137.4 | 214.2 | 228 | 235.3 | 238.2 | 338.5 | 375.5 |
| Sulphate Enoxa | 68.6 | 83.6 | 84.2 | 83 | 87.1 | 94.8 | 101.9 |
| UFH | 60.6 | 66.5 | 76.8 | 77.9 | 98.4 | 103.8 | 136.3 |
| Example 1 | 27.3 | 35 | 133.2 | 170 | 224.8 | 265.9 | 337.2 |
| Example 3 | 28.7 | 43.5 | 108.6 | 104.8 | 217.4 | 302 | 395 |
| Example 4 | 81.5 | 88.2 | 125.9 | 162.7 | 171.7 | 341.1 | 322.7 |
| Comp. Ex. 5 | 45 | 79.5 | 121.4 | 172.5 | 183.6 | 211.6 | 196.7 |

### IN VIVO STUDIES

The tested samples and reference compounds to CD1 mice were administrated in vivo either by i.v. (tail vein) or by i.p. route.

### Effect of pretreatment of animals, by intravenous route, with the test compounds in a model of pulmonary thromboembolism induced by thrombin in mice: mortality studies.

In order to check the antithrombotic properties of the selected heparins that were found active for their anticoagulant properties in vitro, in human plasma, we performed a series of dose-response experiments. Mortality studies, by the i.v. injection of human thrombin (1250U/kg), were carried out two minutes after the injection of the test heparins by using an established animal model (see e.g. P. Gresele, S. Momi, M. Berrettini., G.G. Nenci., H.P. Schwarz., N. Semeraro, M. Colucci. J Clin Invest 1998, 101(3): 667-676; W. Paul, P. Gresele, S. Momi, G. Bianchi, C.P. Page., Br J Pharmacol 1993, 110: 1565-1571). In each experimental session we will compare the test heparins with a low molecular weight heparin (enoxaparin) and with a standard unfractionated heparin (G3000). Data are reported in table 5. All the heparin derivatives tested exerted a dose-dependent protection against thrombin-induced pulmonary thromboembolic mortality.

**Table 5 (Mortality %)**

| | 0 mg/kg | 0.5 mg/kg | 1 mg/kg | 2 mg/kg | 4 mg/kg |
|---|---|---|---|---|---|
| Example 1 | 92 | 52.9 | 48.14 | 35.29 | - |
| Example 3 | 92 | 60 | 66.6 | 33.3 | 20 |
| Example 4 | 92 | 80 | 66.6 | 50 | 30 |
| Comp. Ex. 5 | 92 | 60 | 73.33 | 40 | 26.6 |
| Enoxa | 92 | - | 48 | - | - |
| UFH | 92 | 6.66 | 2 | - | - |

### Effect of pretreatment per os of animals with heparins in a model of pulmonary thromboembolism in mice: mortality studies.

The selected compounds active in vitro and that have shown antithrombotic properties in vivo after i.v. injection, were administered orally to perform thrombin-induced mortality using different doses. Thrombin-induced mortality was carried out 2 hrs after the drug administration.

In one selected experiment thrombin-induced thromboembolism was carried out 4 hrs after the oral administration of the drug (80 mg/kg).

After oral administration, NASSH compounds tested showed activity and reduced significantly moratlity rate in mice. G3000 and enoxaparin, as expected, were totally ineffective up to 80 mg/kg.

The effect of the tested compounds was similar when the thrombotic challenge was carried out 4 hrs after drug administration with a protection observed with G3958 and G3919A.

**Table 6 (Mortality%)**

| | 20 mg/kg | 40 mg/kg | 80 mg/kg |
|---|---|---|---|
| Example 1 | 66.6 | 66.6 | 45 |
| Example 3 | 60 | 68.75 | 60 |
| Comp. Ex. 5 | 60 | 52.63 | 40 |
| ENOXA | 80 | 86.6 | 90 |
| UFH | 80 | 93.3 | 80 |

### Effect of pretreatment of animals with oral heparins in a model of tail transection bleeding time.

Bleeding time was assessed by a tail transection method, adapted from a method previously described for rats (1-3). Briefly, mice orally pretreated two hours before the tail transection bleeding time with the different drugs or their vehicle, were positioned in a special immobilization cage which keeps the tail of the animal steady and immersed in saline thermostated at 37°C. After two minutes the tip of the tail was transected with a razor blade, at a fixed level (2 mm from its end). The tail was immediately reimmersed in thermostated saline, and the time taken to stop bleeding was measured by a chronometer.

The end point is the arrest of bleeding lasting for more than 30 sec. 4 mice per experimental group were tested.

None of the tested compounds did show any prohaemorrhagic effect. Only G3919a and, to a minimal, not significant, extent, G3958, induced a prolongation of the tail transection bleeding time. Under identical conditions unfractionated heparin, by i.v. route 2 min. before the test, prolongs strikingly the bleeding time (>900 sec) at 1 mg/kg (1).

### Effect of pretreatment of animals with oral heparin derivatives on ex vivo coagulation parameters.

In some selected experiments, animals were pretreated with oral heparins at the dose of 80 mg/kg. One hour after treatment mice were anesthetized and blood was collected by cardiac puncture to perform ex vivo measurement, of aPTT, TcT and anti Xa. Data were reported in the following table (tab. 7).

**Tab. 7**

| | **APTT (sec)** | **TcT (sec)** | **Anti Xa (U/ml)** |
|---|---|---|---|
| Example 1 | 25.0 | 21.30 | 0.058 |
| Example 3 | 24.6 | 20.24 | 0.023 |
| Comp. Ex. 5 | 21.6 | 19.76 | 0.073 |
| UFH | 24.7 | 20.2 | 0.064 |
| ENOXA | 23.8 | 18.18 | 0.133 |
| POOL | 24.8 | 22.10 | - |

None of the samples administered by oral route did induce any modification of the coagulative parameters measured (aPTT, TcT, anti Xa).

In order to assess whether a slight residual antithrombin activity could be detected a diluted TcT test was performed, a condition that allows to detect minimal antithrombin activity (Tab. 8).

**Tab 8 - Drugs administered by oral route (dose 80 mg/kg) 2 hrs before the blood sampling.**

| | **TcT(sec)** | **Anti Xa(U/ml)** |
|---|---|---|
| UFH | 32.07 | 0.02 |
| ENOXA | 56.95 | 0.075 |
| Example 1 | 17.8 | 0.035 |
| Comp. Ex. 5 | 20.5 | 0.023 |
| Pool | 21 | - |

### Effect of pretreatment of animals with heparin derivatives by i.v. route on ex vivo coagulation parameters.

The aPTT, TcT and anti Xa ex vivo in plasma of mice treated with G3919A and G3958 (4 mg/kg) 2 min before blood sampling are reported in the following table.

**Tab. 9**

| | **APTT (sec)** | **TcT (sec)** | **Anti Xa(U/ml)** |
|---|---|---|---|
| Example 1 | >300 | >167 | 0.25 |
| Comp. Ex. 5 | >300 | >167 | 1.058 |

Given the maximal prolongation of the TcT observed with 4 mg/kg hypersulphated heparins, a lower dose was tested. Here too, in order to assess whether a slight residual antithrombin activity could be detected a diluted TcT test was performed, a condition that allows to detect minimal antithrombin activity (Tab. 10).

**Tab. 10 - Drugs administered by i.v. route 2 min (dose 2 mg/kg) before blood sampling (TcT and anti Xa diluted)**

| | **TcT (sec)** | **Anti Xa (U/ml)** |
|---|---|---|
| UFH | 167 | 1.25 |
| ENOXA | 167 | 2.02 |
| Comp. Ex. 5 | 10.12 | 7.37 |
| Example 1 | 12.00 | 1.08 |
| Pool | 21.80 | - |

## Claims

1. Pharmaceutical composition for use in therapy by oral administration comprising N-acetylated, oversulfated heparin **characterized by** the following parameters: percentage of N-acetylation >50%, degree of sulfation >2.5, weight average molecular weight comprised between 1,600 and 10,000, wherein the composition is not in the form of gastro-resistant capsules, tablets, lozenges, pills or liposomes.

2. Pharmaceutical composition for use according to claim 1 wherein the composition does not comprise a carrier molecule or a carrier system.

3. Pharmaceutical composition for use according to claims 1-2, wherein the percentage of N-acetylation is higher than or equal to 70%.

4. Pharmaceutical composition for use according to claims 1-3, wherein the N-acetylated oversulfated heparin has a Mw comprised between 4,000 and 8,000

5. Pharmaceutical composition for use according to claims 1-4 wherein the degree of sulfation is higher than 2.8

6. Pharmaceutical composition for use according to claim 6 wherein the degree of sulfation is higher than 3.

## Patentansprüche

1. Pharmazeutische Zusammensetzung zur therapeutischen Verwendung durch orale Verabreichung enthaltend N-acetyliertes, übersulfatiertes Heparin **gekennzeichnet durch** die folgenden Parameter: Prozentsatz der N-Acetylierung > 50%, Sulfatierungsgrad > 2,5, Gewichtsmittel des Molekulargewichts zwischen 1.600 und 10.000, worin die Zusammensetzung nicht in Form von magensaftresistenten Kapseln, Tabletten, Pastillen, Pillen oder Liposomen vorliegt.

2. Pharmazeutische Zusammensetzung zur Verwendung gemäß Anspruch 1, worin die Zusammensetzung kein Trägermolekül oder Trägersystem enthält.

3. Pharmazeutische Zusammensetzung zur Verwendung gemäß Ansprüchen 1-2, worin der Prozentsatz der N-Acetylierung größer oder gleich 70% ist.

4. Pharmazeutische Zusammensetzung zur Verwendung gemäß Ansprüchen 1-3, worin das N-acetylierte, übersulfatierte Heparin ein Mw zwischen 4.000 und 8.000 aufweist.

5. Pharmazeutische Zusammensetzung zur Verwendung gemäß Ansprüchen 1-4, worin der Sulfatierungsgrad größer als 2,8 ist.

6. Pharmazeutische Zusammensetzung zur Verwendung gemäß Anspruch 6, worin der Sulfatierungsgrad größer als 3 ist.

## Revendications

1. Composition pharmaceutique pour une utilisation en thérapie par administration orale, comprenant de l'héparine sursulfatée, N-acétylée, **caractérisée par** les paramètres suivants : pourcentage de N-acétylation > 50 %, degré de sulfatation > 2,5, masse moléculaire moyenne en poids comprise entre 1 600 et 10 000, la composition n'étant pas sous la forme de capsules, comprimés, pastilles, pilules ou liposomes gastro-résistants.

2. Composition pharmaceutique pour une utilisation selon la revendication 1, dans laquelle la composition ne comprend pas de molécule support ou de système porteur.

3. Composition pharmaceutique pour une utilisation selon l'une des revendications 1 et 2, dans laquelle le pourcentage de N-acétylation est supérieur ou égal à 70 %.

4. Composition pharmaceutique pour une utilisation selon l'une des revendications 1 à 3, dans laquelle l'héparine sursulfatée N-acétylée a une Mw comprise entre 4 000 et 8 000.

5. Composition pharmaceutique pour une utilisation selon l'une des revendications 1 à 4, dans laquelle le degré de sulfatation est supérieur à 2,8.

6. Composition pharmaceutique pour une utilisation selon la revendication 6, dans laquelle le degré de sulfatation est supérieur à 3.
